# EUROPEAN PATENT APPLICATION

(11) **EP 4 516 856 A1**
(43) Date of publication of application: **05.03.2025**
(21) Application number: 23194651.8
(22) Date of filing: 31.08.2023
(51) Int. Cl.: C08L 53/02, C08K 3/08, C08K 3/04, C08L 51/00, A61B 5/0245

(54) **MASS PRODUCIBLE MATERIAL FORMULATION FOR SOFT, LOW-IMPEDANCE SKIN CONTACT IN DRY BIOPOTENTIAL ELECTRONICS**

(71) Applicant: IDUN Technologies AG, 8152 Opfikon (CH); EMPA Eidgenössische Materialprüfungs- und Forschungsanstalt, 8600 Dübendorf (CH)
(72) Inventor: THIELEN, Moritz, 8152 Glattpark (Opfikon) (CH); JUNKER, Katja, 8152 Glattpark (Opfikon) (CH); CLEMENS, Frank Jörg, 8600 Dübendorf (CH); ECKEY, Louisa Marie, 8600 Dübendorf (CH); GEORGOPOULOU-PAPADONIKOLAKI, Antonia, 8600 Dübendorf (CH)
(74) Representative: Vossius & Partner Patentanwälte Rechtsanwälte mbB

(57) **Abstract**

The present disclosure relates to a composition comprising (A) a thermoplastic elastomer containing groups derived from a dicarboxylic acid anhydride containing an aliphatic C=C double bond, (8) carbon black, and (C) silver particles, to a method of manufacturing the composition, to a molded article and an electronic device obtainable by molding the composition, to the use of the molded article or the electronic device for measuring an electrophysiological signal, and to a method of measuring an electrophysiological signal in which the molded article or the electronic device is used.

## Description

### Field of the disclosure

The present disclosure relates to a composition comprising (A) a thermoplastic elastomer containing groups derived from a dicarboxylic acid anhydride containing an aliphatic C=C double bond, (B) carbon black, and (C) silver particles, to a method of manufacturing the composition, to a molded article and an electronic device obtainable by molding the composition, to the use of the molded article or the electronic device for measuring an electrophysiological signal, and to a method of measuring an electrophysiological signal in which the molded article or the electronic device is used.

### Background

In many medical diagnostic systems skin surface electrodes play a major role in detecting certain electrophysiological signals including, for example, electrocardiography (ECG), electromyography (EMG), and electroencephalography (EEG).

The function of skin surface electrodes is dependent on a multitude of mechanisms, such as electrode material, the electrolyte applied to the electrode, body location, and skin properties. Typically, a physiological ionic current is converted into an electronic current receivable and processable by the associated instrumentation, e.g. by use of a processor. In order to lower the impedance between the surface of the electrode and the surface of the skin contacted by the electrode: an electrolyte gel containing chloride ions has previously been applied to provide adequate ionic contact with the skin. However, the use of such gel is often a discomfort to the patient and the gel usually has poor consistency, dries quickly and tends to spread, which can lead to reduced signal quality due to interaction with neighboring electrodes.

The attachment of surface electrodes on skin is time-consuming and therefore costly. In particular for high-performance measurements in which multiple electrodes are used, ease of attachment to the skin is thus required.

It is desired to have electrodes that can be applied directly to the skin without the need for gel or abrasion of skin. Furthermore, in view of environmental concerns, reusability of such electrodes is desired. Such electrodes that can easily be applied to the skin, removed and reattached to skin furthermore open multiple avenues with respect to user-friendly recording and analysis of vital physiological functions, not only in medical environments but also in long-term and mobile applications during every-day life. For example, such electrodes may be beneficially included in ear-tips used for consumer electronics and serve to measure vital physiological signals in order to improve and adapt work-out procedures, or monitor sleep, while at the same time ensuring that life-threatening conditions are detected as early as possible and/or avoided.

### Summary

In view of the state of the art, it is an objective of the present disclosure to provide a flexible and conductive composition having excellent conformability - with skin as well as having low resistance and skin contact impedance.

This objective can be achieved by the composition of the present disclosure which comprises (A) a thermoplastic elastomer containing groups derived from a dicarboxylic acid anhydride containing an aliphatic C=C double bond, (B) carbon black, and (C) silver particles. The composition preferably also contains (D) a thermoplastic elastomer containing essentially no groups derived from a dicarboxylic acid anhydride containing an aliphatic C=C double bond.

According to the disclosure, the composition is preferably prepared by a process comprising providing molten (A) (and (D)), and slowly adding a mixture of (B) and (C).

The present disclosure furthermore relates to a molded article and an electronic device obtainable by molding the composition. Such molded article or electronic device is preferably in the form of a pad or an eartip, or part of an eartip (including any form of stabilizing structure that helps to stabilize earbuds in the ear, e.g. in the concha of the ear), earclip, watch, ring, glasses, underwear, belt, hat, shirt, chest strap, headband, helmet, glove, socks, shoe, mouse, game controller, steering wheel (such as for a car, truck, train, plane or boat), remote control (such as for a TV, drone, car or boat), bioimpedance scale, or any other device commonly being used in contact with human skin.

In addition, the disclosure concerns the use of the molded article or electronic device in one or more selected from an electrocardiogram (ECG), an electromyogram (EMG), an electroencephalogram (EEG), a galvanic skin response (GSR), an electrooculogram (EOG), a body temperature, a pulse, a blood pressure, a body movement, and any combination thereof.

The present disclosure also concerns a method of measuring an electrophysiological signal including the steps of (i) applying one or more, preferably two or more, of molded articles according to any one of claims 30 to 35 or electronic devices according to any one of claims 36 to 38 to part of the skin of an animal, and (ii) measuring electric voltage.

### Definitions

The term "dicarboxylic acid anhydride containing an aliphatic C=C double bond" is not particularly limited and may be any anhydride of a dicarboxylic acid which contains an aliphatic C=C double bond. Preferably the anhydride is an anhydride in which the two carboxylic acid groups form a cyclic anhydride. Preferably, the two carboxylic acid groups forming the anhydride are connected to neighboring carbon atoms. These neighboring carbon atoms are preferably the carbon atoms of the aliphatic C=C double bond. The term "dicarboxylic acid anhydride containing an aliphatic C=C double bond" more preferably refers to maleic acid anhydride.

The term "derived from a dicarboxylic acid anhydride containing an aliphatic C=C double bond" preferably means that a (co)polymer contains repeating units which are obtained by copolymerizing with a dicarboxylic acid anhydride containing an aliphatic C=C double bond. The copolymerizing with the dicarboxylic acid anhydride containing an aliphatic C=C double bond is preferably graft polymerization with the dicarboxylic acid anhydride containing an aliphatic C=C double bond. Thus, typically, unit resulting from polymerization with the dicarboxylic acid anhydride containing an aliphatic C=C double bond is present not in the polymer backbone. Preferably, the unit of the dicarboxylic acid anhydride containing an aliphatic C=C double bond is present as side chains and/or at the ends of the polymer chains. An example of a "group derived from a dicarboxylic acid anhydride containing an aliphatic C=C double bond" is a group having the following formula: 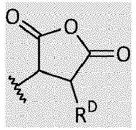 wherein the wavy line indicates the attachment point to the remainder of the polymer and R^{D} indicates hydrogen or a C₁₋₆ alkyl group, preferably hydrogen or methyl, more preferably hydrogen.

It is furthermore envisaged, that two or more residues derived from dicarboxylic acid anhydride containing an aliphatic C=C double bond may be bound directly to each other, such as in the following formula: 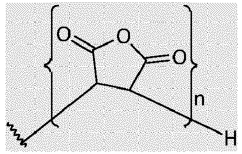 wherein n is preferably an integer from 2 to 20, more preferably 2 to 10.

The term "high shear", as used herein, preferably refers to applying shear to the material during the fabrication of the material mixture and subsequent processing. High shear conditions influence the distribution of particles in the material, and therefore the electrical properties of it. High shear conditions lead to a more homogeneous particle distribution and increase the electrical resistivity in the material (DOI 10.1007/978-3-319-28117-9_32). High shear conditions are characterized in this case by mixing speeds higher than or equal to 220 rpm in the mixing zone of, e.g, a buss kneader, with an output of at least 20 kg material/h during the fabrication of the material mixture.

The term "low shear", as used herein, typically refers to conditions with mixing speeds lower than 220 rpm in the mixing zone of, e.g., a Buss Kneader, with an output of lower than 20 kg material/h. They typically lead to slight inhomogeneities in the material, that generally lead to lower resistivities, which are desired in this case.

### Description of the Figures

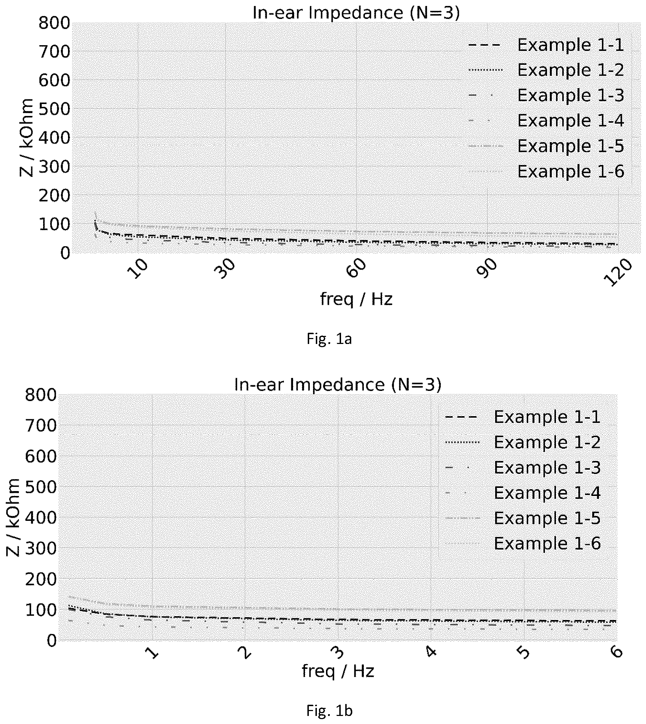

- **Fig. 1a and b**: show the mean in-ear impedance measured of Example 1-1, 1-2, 1-3, 1-4, 1-5 and 1-6, according to the description in Example 1. Fig. 1a shows results between 0.1 and 120 Hz, while Fig. 1b shows the same data as Fig. 1a, but from 0.1 Hz to 6 Hz. Data were recorded from left and right ears of three subjects

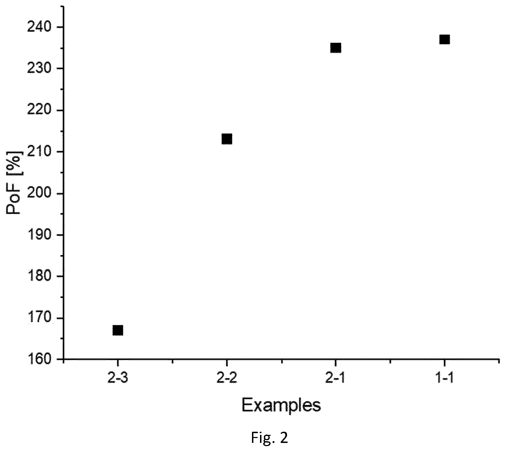

- **Fig. 2**: shows the comparison between the point of fracture of the compositions from Example 1-1, 2-1, 2-2 and 2-3 with different amounts of the component (A) SCONA TSKD 9103.

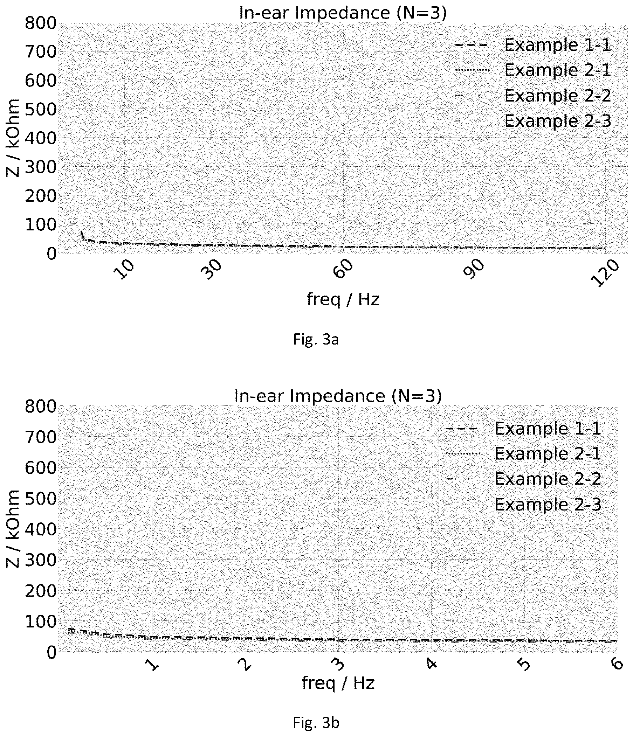

- **Fig. 3a and b**: show a comparison of the mean in-ear impedance for Example 1-1 with 20 wt.-% (A), with Example 2-1 with 15 wt.-% (A), Example 2-2 with 10 wt.-% of (A) and Example 2-3 without addition of (A). Fig. 3a shows results between 0.1 and 120 Hz, while Fig. 3b shows the same data as Fig. 3a, but from 0.1 Hz to 6 Hz. Data were recorded from left and right ears of three subjects.

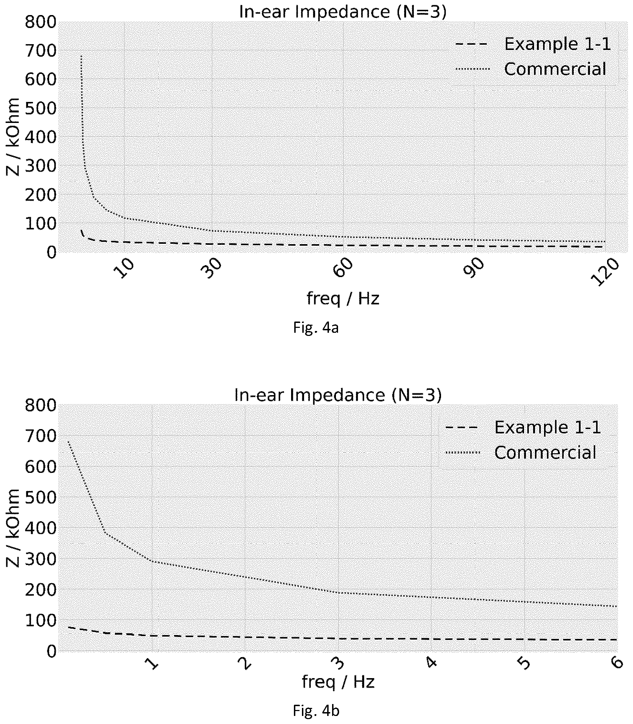

- **Fig. 4a and b**: show a comparison of the mean in-ear impedance for the invention (Example 1-1) and a commercially available material (Commercial). Fig. 4a shows results between 0.1 and 120 Hz, while Fig. 4b shows the same data as Fig. 4a, but from 0.1 Hz to 6 Hz. Data were recorded from left and right ears of three subjects.

### Detailed description

In the prior art, it has generally been difficult to simultaneously achieve good skin compatibly, flexibility, reusability and low skin contact impedance. The present inventors surprisingly found that these effects can be achieved by a composition comprising (A) a thermoplastic elastomer containing groups derived from a dicarboxylic acid anhydride containing an aliphatic C=C double bond, (B) carbon black, and (C) silver particles. Without wishing to be bound by theory, it is assumed that the thermoplastic elastomer containing groups derived from a dicarboxylic acid anhydride containing an aliphatic C=C double bond provides not only for flexibility but also for good compatibility with the carbon black and the silver particles. Furthermore, by using both carbon black and the silver particles low skin contact impedance can be achieved without detrimental effects on the flexibility and reusability.

### The composition

As set out above, the present disclosure relates to a composition comprising (A) a thermoplastic elastomer containing groups derived from a dicarboxylic acid anhydride containing an aliphatic C=C double bond, (B) carbon black, and (C) silver particles. The composition preferably also contains (D) a thermoplastic elastomer containing essentially no groups derived from a dicarboxylic acid anhydride containing an aliphatic C=C double bond.

In the present disclosure, the dicarboxylic acid anhydride containing an aliphatic C=C double bond is preferably maleic acid anhydride.

In particular, if component (D) is contained, (A) is usually contained in the composition in a range of from 5 to 50 wt-%, preferably 8 to 40 wt-%, more preferably 10 to 30 wt.-%, even more preferably 15 to 25 wt.-%, still more preferably 17 to 23 wt.-%, most preferably 19 to 21 wt.-%, such as 19 wt.-%, 20 wt.-%, or 21 wt.-%, relative to 100 wt.-% of the composition.

If component (D) is not contained, (A) is usually contained in the composition in a range of from 10 to 90 wt-%, preferably 15 to 70 wt-%, more preferably 25 to 70 wt.-%, even more preferably 25 to 65 wt.-%, still more preferably 30 to 60 wt.-%, still even more preferably 30 to 50 wt.-%, such as 30 wt.-%, 31 wt.-%, 32 wt.-%, 33 wt.-%, 34 wt.-%, 35 wt.-%, 36 wt.-%, 37 wt.-%, 38 wt.-%, 39 wt.-%, 40 wt.-%, 41 wt.-%, 42 wt.-%, 43 wt.-%, 44 wt.-%, 45 wt.-%, 46 wt.-%, 47 wt.-%, 48 wt.-%, 49 wt.-%, or 50 wt.-%, most preferably 33 to 44 wt.-%, such as 37 wt.-%, 38 wt.-%, 39 wt.-%, 40 wt.-%, 41 wt.-%, or 42 wt.-%, (such as preferably 38 wt.-%, 39 wt.-%, or 40 wt.-%) relative to 100 wt.-% of the composition.

It is preferred that the total polymer content in the composition be in a range of from 10 to 90 wt-%, preferably 15 to 70 wt-%, more preferably 25 to 70 wt.-%, even more preferably 25 to 65 wt.-%, still more preferably 30 to 60 wt.-%, still even more preferably 30 to 50 wt.-%, such as 30 wt.-%, 31 wt.-%, 32 wt.-%, 33 wt.-%, 34 wt.-%, 35 wt.-%, 36 wt.-%, 37 wt.-%, 38 wt.-%, 39 wt.-%, 40 wt.-%, 41 wt.-%, 42 wt.-%, 43 wt.-%, 44 wt.-%, 45 wt.-%, 46 wt.-%, 47 wt.-%, 48 wt.-%, 49 wt.-%, or 50 wt.-%, most preferably 33 to 44 wt.-%, such as 37 wt.-%, 38 wt.-%, 39 wt.-%, 40 wt.-%, 41 wt.-%, or 42 wt.-%, (such as preferably 38 wt.-%, 39 wt.-%, or 40 wt.-%) relative to 100 wt.-% of the composition.

The total content of groups derived from a dicarboxylic acid anhydride containing an aliphatic C=C double bond in the composition is typically at least 0.05 wt.-%, preferably at least 0.1 wt.-%, more preferably at least 0.2 wt.-%, or even at least 0.25 wt.-%, expressed as the weight of the atoms that were part of the dicarboxylic acid anhydride containing an aliphatic C=C double bond relative to 100 wt.-% of the composition. The total content of groups derived from a dicarboxylic acid anhydride containing an aliphatic C=C double bond in the composition is typically 5 wt.-% or less, preferably 2 wt.-% or less, more preferably 1 wt.-% or less, or even 0.5 wt.-% or less, expressed as the weight of the atoms that were part of the dicarboxylic acid anhydride containing an aliphatic C=C double bond relative to 100 wt.-% of the composition.

It is to be understood that the groups derived from a dicarboxylic acid anhydride containing an aliphatic C=C double bond in the composition typically refer to the groups derived from a dicarboxylic acid anhydride containing an aliphatic C=C double bond that are present in component (A).

The content of (B) is typically in a range of from 1 to 40 wt-%, preferably 2 to 35 wt-%, more preferably 3 to 30 wt.-%, even more preferably 5 to 30 wt.-%, still more preferably 5 to 25 wt.-%, still even more preferably 10 to 20 wt.-%, such as 10 wt.-%, 11 wt.-%, 12 wt.-%, 13 wt.-%, 14 wt.-%, 15 wt.-%, 16 wt.-%, 17 wt.-%, 18 wt.-%, 19 wt.-%, or 20 wt.-%, (preferably 14 wt.-%, 15 wt.-%, 16 wt.-%, 17 wt.-%, or 18 wt.-%, more preferably 15 wt.-%, 16 wt.-% or 17 wt.-%) relative to 100 wt.-% of the composition.

Generally, the composition contains (C) in a range of from 10 to 90 wt-%, preferably 20 to 70 wt-%, more preferably 25 to 65 wt.-%, even more preferably 30 to 60 wt.-%, still more preferably 36 to 60 wt.-%, still even more preferably 36 to 57 wt.-%, such as 36 wt.-%, 37 wt.-%, 38 wt.-%, 39 wt.-%, 40 wt.-%, 41 wt.-%, 42 wt.-%, 43 wt.-%, 44 wt.-%, 45 wt.-%, 46 wt.-%, 47 wt.-%, 48 wt.-%, 49 wt.-%, 50 wt.-%, 51 wt.-%, 52 wt.-%, 53 wt.-%, 54 wt.-%, 55 wt.-%, 56 wt.-%, or 57 wt.-%, relative to 100 wt.-% of the composition. Particularly preferred are 40 wt.-%, 41 wt.-%, 42 wt.-%, 43 wt.-%, 44 wt.-%, 45 wt.-%, 46 wt.-%, 47 wt.-%, 48 wt.-%, 49 wt.-%, or 50 wt.-%; more preferred are 42 wt.-%, 43 wt.-%, 44 wt.-%, 45 wt.-%, 46 wt.-%, or 47 wt.-%, such as 43 wt.-%, 44 wt.-%, 45 wt.-%, or 46 wt.-%.

Lower concentrations of compound (C) can be used, but typically lead to a lower signal-to-noise ratio, in particular at frequencies below 4 Hz (also known as delta waves, e.g. in EEG). These are highly relevant for different biopotential recordings such as ECG, EEG and galvanic skin response. Such delta waves are of interest especially for monitoring sleep. In view of the costs of high amounts of silver, it may thus be preferable to use a lower silver content in applications where frequencies below 4 Hz are not of particular interest. Thus, especially in those cases, the composition may contain (C) in a range of from 10 to 90 wt-%, preferably 10 to 70 wt-%, more preferably 10 to 65 wt.-%, even more preferably 15 to 36 wt.-%, such as 15 wt.-%, 16 wt.-%, 17 wt.-%, 18 wt.-%, 19 wt.-%, 20 wt.-%, 21 wt.-%, 22 wt.-%, 23 wt.-%, 24 wt.-%, 25 wt.-%, 26 wt.-%, 27 wt.-%, 28 wt.-%, 29 wt.-%, 30 wt.-%, 31 wt.-%, 32 wt.-%, 33 wt.-%, 34 wt.-%, 35 wt.-%, or 36 wt.-%, relative to 100 wt.-% of the composition.

Compound (D) is not an essential feature of the present disclosure but may lead to improved flexibility. For example, the composition may contain (D) in a range of from 5 to 50 wt-%, preferably 5 to 30 wt.-%, more preferably 10 to 30 wt.-%, even more preferably 10 to 25 wt.-%, still more preferably 12 to 24 wt.-%, most preferably 13 to 24 wt.-%, such as 13 wt.-%, 14 wt.-%, 15 wt.-%, 16 wt.-%, 17 wt.-%, 18 wt.-%, 19 wt.-%, 20 wt.-%, 21 wt.-%, 22 wt.-%, 23 wt.-%, or 24 wt.-%, relative to 100 wt.-% of the composition.

In order to achieve excellent flexibility and reusability, the ratio of (B) to (C) (each in wt.-%) is typically in the range of from 0.01 to 50, preferably 0.05 to 20, more preferably 0.1 to 10, even more preferably 0.2 to 5, still more preferably 0.2 to 2, even still more preferably 0.2 to 1, such as 0.2 to 0.5, preferably 0.3 to 0.4, more preferably 0.33 to 0.39, such as 0.33, 0.34, 0.35, 0.36, 0.37, 0.38 or 0.39, most preferably 0.36.

The total content of (A), (B), (C) and (D) is typically at least 70 wt.-%, preferably at least 75 wt.-%, more preferably at least 80 wt.-%, even more preferably at least 90 wt.-%, still more preferably at least 95 wt.-%, or even at least 96 wt.-%, at least 97 wt.-%, at least 98 wt.-%, at least 99 wt.-%, at least 99.5 wt.-%, or at least 99.9 wt.-%, relative to 100 wt.-% of the composition.

The total content of (B) and (C) is typically in a range of from 20 to 90 wt-%, preferably 30 to 80 wt-%, more preferably 35 to 80 wt.-%, even more preferably 40 to 75 wt.-%, still more preferably 50 to 70 wt.-%, still even more preferably 50 to 67 wt.-%, such as 50 wt.-%, 51 wt.-%, 52 wt.-%, 53 wt.-%, 54 wt.-%, 55 wt.-%, 56 wt.-%, 57 wt.-%, 58 wt.-%, 59 wt.-%, 60 wt.-%, 61 wt.-%, 62 wt.-%, 63 wt.-%, 64 wt.-%, 65 wt.-%, 66 wt.-%,or 67 wt.-%, relative to 100 wt.-% of the composition.

The ratio of (A) to the sum of (B) and (C) (each in wt.-%) is typically in the range of from 0.01 to 50, preferably 0.05 to 20, more preferably 0.1 to 10, even more preferably 0.1 to 5, still more preferably 0.2 to 2. If (D) is present, the ratio of (A) to the sum of (B) and (C) (each in wt.-%) is even still more preferably 0.2 to 0.5, such as 0.3 to 0.4. If (D) is absent, the ratio of (A) to the sum of (B) and (C) (each in wt.-%) is even still more preferably 0.5 to 1.0, such as 0.5 to 0.8.

If (D) is present, the ratio of the sum of (A) and (D) to the sum of (B) and (C) (each in wt.-%) is even still more preferably 0.5 to 1.0, such as 0.5 to 0.8.

The ratio of (A) to (C) (each in wt.-%) is typically in the range of from 0.01 to 50, preferably 0.05 to 20, more preferably 0.1 to 10, even more preferably 0.1 to 5, still more preferably 0.2 to 2. If (D) is present, the ratio of (A) to (C) (each in wt.-%) is even still more preferably 0.3 to 0.7, such as 0.4 to 0.6. If (D) is absent, the ratio of (A) to (C) (each in wt.-%) is even still more preferably 0.6 to 1.4, such as 0.8 to 1.2, most preferably 0.8 to 1.0, such as 0.85, 0.86, 0.87, 0.88, 0.89, 0.90, 0.91, 0.92 or 0.93, among which 0.88 is preferred.

The composition of the present disclosure may furthermore contain (E) paraffin, polyethylene and/or polypropylene. The total content of component (E), i.e. the total content of paraffin, polyethylene and polypropylene, relative to 100 wt.-% of the composition, is preferably 20 wt.-% or less, more preferably 10 wt.-% or less, even more preferably 5 wt.-% or less, still more preferably 2 wt.-% or less. If (E) is contained, it is preferred that the total content of (A), (B), (C), (D) and (E) be at least 70 wt.-%, preferably at least 75 wt.-%, more preferably at least 80 wt.-%, even more preferably at least 90 wt.-%, still more preferably at least 95 wt.-%, or even at least 96 wt.-%, at least 97 wt.-%, at least 98 wt.-%, at least 99 wt.-%, at least 99.5 wt.-%, or at least 99.9 wt.-%, relative to 100 wt.-% of the composition.

With the composition of the present invention, low resistivities, such as 10 Ohm*m or less, preferably 5 Ohm*m or less, more preferably 2 Ohm*m or less, even more preferably 1 Ohm*m or less, 0.5 Ohm*m or less, 0.3 Ohm*m or less, 0.2 Ohm*m or less, or even 0.1 Ohm*m or less can be achieved. Resistivity can be measured with a digital source meter 2450 from KEITHELY, USA. The resistivity-measurement is usually done three times on extruded fibers with a diameter of 0.5 mm and a measurement length of 5 cm. The resistivity is calculated by the dimensions of the sample and the resistance of the fiber.

With molded articles of the present invention, low skin contact impedances, such as 500 kOhm or less, preferably 400 kOhm or less, more preferably 300 kOhm or less, even more preferably 200 kOhm or less, or even 100 kOhm or less can be achieved in the frequency range between 0.1 and 120 Hz. Skin contact impedance can be measured in the outer ear canal of human test subjects with an impedance analyzer, such as a PalmSens4 impedance analyzer (PaImSens B.V., Netherlands) using a three-electrode setup,. The skin contact impedance is typically measured on at least three human subjects, three times in each ear. For a better reproducibility of impedance values measured on human skin, the skin is preferably to be cleaned (preferably detergent and water) and a 10 minute long stablilization time of the molded articles before the start of the impedance measurement is to be allowed.

The thermoplastic elastomer containing groups derived from a dicarboxylic acid anhydride containing an aliphatic C=C double bond (A)

The thermoplastic elastomer containing groups derived from a dicarboxylic acid anhydride containing an aliphatic C=C double bond (A) may constitute the main part of the polymer content in the composition of the present disclosure, such as 50 wt.-% or more, 60 wt.-% or more, 70 wt.-% or more, relative to the total polymer content of the composition. The thermoplastic elastomer containing groups derived from a dicarboxylic acid anhydride containing an aliphatic C=C double bond (A) preferably constitutes 45 to 69 wt.-%, more preferably 47 to 53 wt.-%, of the polymer content in the composition of the present disclosure, relative to the total polymer content of the composition. Component (A) can also be used in combination with component (D), for example as a mixture of components (A) and (D).

Component (A) is preferably a block copolymer. More preferably, component (A) is a block copolymer of styrene with one or more selected from ethylene, butylene, propylene and isoprene, which further contains groups derived from a dicarboxylic acid anhydride containing an aliphatic C=C double bond. Component (A) is more preferably a block copolymer of styrene and butylene, optionally with one or both selected from propylene and isoprene, which further contains groups derived from a dicarboxylic acid anhydride containing an aliphatic C=C double bond.

It is to be understood that the Component (A) can also be a partially hydrogenated styrene-block-copolymer. The hydrogenation preferably refers to the hydrogenation of aliphatic C=C double bonds, but not aromatic C=C double bonds. In view of oxidation stability, it is preferred that at least 50 %, preferably at least 60 %, more preferably at least 70 %, even more preferably at least 80 %, or even at least 90 % of all aliphatic C=C double bonds are hydrogenated. In other words, hydrogenated block copolymers typically have a residual aliphatic double bond content of 0.5 to 20%, based on the content present before hydrogenation. Typically less than 20 mol-%, preferably less than 12 mol-%, more preferably less than 5 mol-%, even more preferably less than 3 mol-%, still more preferably less than 2 mol-%, less than 1 mol-%, or even less than 0.5 mol-% of the aliphatic carbon atoms in component (A) are carbon atoms forming a C=C double bond.

As used herein, the term styrene-ethylene-butylene-styrene (SEBS), preferably includes any hydrogenated or partially hydrogenated styrene/butadiene-styrene (SBS) copolymers.

The expression "contains groups derived from a dicarboxylic acid anhydride containing an aliphatic C=C double bond" preferably means that the copolymer is graft polymerized with a dicarboxylic acid anhydride containing an aliphatic C=C double bond.

Component (A) typically contains at least 0.1 wt.-%, preferably at least 0.2 wt.-%, more preferably at least 0.5 wt.-%, even more preferably at least 1.0 wt.-%, or even at least 1.2 wt.-%, groups derived from a dicarboxylic acid anhydride containing an aliphatic C=C double bond, relative to the total weight of (A). Component (A) preferably contains 10 wt.-% or less, preferably 5 wt.-% or less, more preferably 3 wt.-% or less, even more preferably 2 wt.-% or less, or even 1.5 wt.-% or less, groups derived from a dicarboxylic acid anhydride containing an aliphatic C=C double bond, relative to the total weight of (A). The content of groups derived from a dicarboxylic acid anhydride containing an aliphatic C=C double bond can be determined by titration.

Component (A) can also be used in combination with component (D). It is to be understood that the content of groups derived from a dicarboxylic acid anhydride containing an aliphatic C=C double bond in component (A) can be chosen depending on the amount of Component (D). Thus, if Component (D) is present, the amount of groups derived from a dicarboxylic acid anhydride containing an aliphatic C=C double bond in component (A) may be higher than in a case where no component (D) is present. In other words, if component (D) is used in addition to component (A), it is preferred that the total of component (A) and component (D) contains at least 0.1 wt.-%, preferably at least 0.2 wt.-%, more preferably at least 0.3 wt.-%, even more preferably at least 0.5 wt.-%, such as preferably 0.5 to 0.9 wt.-%, more preferably 0.6 to 0.9 wt.-%, groups derived from a dicarboxylic acid anhydride containing an aliphatic C=C double bond, relative to the total weight of (A) and (D).

It is preferred that (A) contains less than 15 wt.-%, preferably less than 10 wt.-%, more preferably less than 8.0 wt.-%, even more preferably less than 6.0 wt.-%, still even more preferably less than 4.0 wt.-%, or even less than 2.0 wt.-%, groups derived from a dicarboxylic acid anhydride containing an aliphatic C=C double bond, relative to the total weight of (A). If component (D) is used in addition to component (A), it is preferred that the total of component (A) and component (D) contains less than 15 wt.-%, preferably less than 10 wt.-%, more preferably less than 8.0 wt.-%, even more preferably less than 6.0 wt.-%, still even more preferably less than 4.0 wt.-%, or even less than 2.0 wt.-%, such as less than 1.5 wt.-%, groups derived from a dicarboxylic acid anhydride containing an aliphatic C=C double bond, relative to the total weight of (A) and (D).

If component (D) is used in addition to component (A), it is preferred that (A) contains less than 20 wt.-%, preferably less than 15 wt.-%, more preferably less than 10 wt.-%, even more preferably less than 8.0 wt.-%, still even more preferably less than 6.0 wt.-%, or even less than 4.0 wt.-%, such as less than 2.5 wt.-%, groups derived from a dicarboxylic acid anhydride containing an aliphatic C=C double bond, relative to the total weight of (A).

Component (A) is preferably a styrene-block-copolymer or a hydrogenated styrene-block-copolymer, obtainable by
(i) polymerizing
   - styrene, and
   - at least one selected from ethylene, butylene, propylene and isoprene,
(ii) optionally hydrogenating the copolymer, and
(iii) graft polymerization of the copolymer or hydrogenated copolymer with a dicarboxylic acid anhydride containing an aliphatic C=C double bond.

Component (A) is, for example, obtainable by reacting the copolymer with maleic acid anhydride and optionally an initiator, such as benzoyl peroxide or azobisisobutyronitrile, preferably at a temperature of 40 to 100°C, more preferably 50 to 90°C, preferably using solid phase grafting using a twin-screw device, such as high-shear solid phase grafting, typically as set out in WO 93/24537.

The carboxylic acid anhydride containing an aliphatic C=C double bond is preferably maleic acid anhydride. Thus, (A) is preferably a maleic acid anhydride modified styrene-block-copolymer selected from maleic acid anhydride modified styrene-ethylene-butylene-styrene (SEBS), maleic acid anhydride modified styrene/butadiene-styrene (SBS), maleic acid anhydride modified styrene-ethylene/propylene-styrene (SEPS), maleic acid anhydride modified styrene-isoprene-styrene (SIS), or a hydrogenated copolymer of these.

Thus, (A) is preferably a styrene-ethylene/butylene-styrene-block-copolymer, preferably a styrene-ethylene-butylene-styrene-block-copolymer which has been modified with maleic acid anhydride, such as a copolymer obtainable by polymerization at least styrene, maleic acid anhydride and ethylene or butylene.

Thermally very stable graft products, which also have improved mechanical properties and higher transparency and flowability, are obtained by grafting maleic acid anhydride onto block copolymers with a high 1,2-configuration and a high degree of hydrogenation, such as in particular SEBS. Under melt blending conditions, preferably in an extruder using a peroxidic initiator and from about 0.2 to 5.0% maleic acid anhydride, grafting occurs predominantly at the tertiary carbon atoms of the EB segments. Solution grafting is rather uneconomical, due to the need of using large amounts of solvents which can lead to disadvantages regarding disposal and work-place safety. Thus, melt grafting, especially based on selectively hydrogenated styrene-diene block copolymers as backbone polymers, is technically used on a larger scale.

As shown in WO 93/24537 (A1), melt grafting can, e.g. be conducted at polymerization temperatures between 40 and 100 °C, preferably between 50 and 90 °C, in dry powdery and/or crumbly and/or flaky and/or granular reaction.

Styrene, C₄₋₁₆ olefins or dienes and C₁₋₈ alkyl esters of acrylic acid or methacrylic acid can be used as particularly suitable comonomers.

Suitable initiators are the known organic peroxidic and/or diazo group-containing compounds with a ten-hour half-life temperature (10h-HWT, measured in 1.0-m-benzene solution) between 40 and 80 °C, in particular peroxidicarbonates, perneodecanoates and diacyl peroxides.

### The carbon black (B)

The carbon black (B) typically has a BET surface in the range of from 10 to 1500 m²/g, preferably 10 to 1000 m²/g, more preferably 20 to 900 m²/g, even more preferably 25 to 600 m²/g, still more preferably 25 to 400 m²/g, even still more preferably 30 to 200 m²/g, most preferably 30 to 100 m²/g or even 50 to 90 m²/g, as measured according to ASTM D3037-89.

Component (B) preferably has a pour density measured according to ASTM D1513 of 130 to 210, preferably 150 to 190, more preferably 160 to 180 g/l. The oil absorption number measured according to ASTM D2414 is typically 170 to 210, preferably 180 to 200, more preferably 185 to 195 ml/100g. The grit 325 mesh residue according to ASTM D1514 of component (B) is typically 50 wt.-ppm or less, preferably 20 wt.-ppm or less, more preferably 10 wt.-ppm or less, even more preferably 5 wt.-ppm or less.

Examples of suitable carbon blacks as component (B) include, but are not limited to, "Ensaco 260G" or "Ensaco 360G" by Imerys.

### The silver particles (C)

The silver particles (C) typically have a median particle diameter in the range of from 0.1 to 20 µm, preferably 0.2 to 15 µm, more preferably 0.3 to 13 µm, even more preferably 0.4 to 12 µm, still more preferably 0.5 to 10 µm, even still more preferably 1 to 10 µm, most preferably 2 to 9 µm, as measured by laser granulometry, preferably using laser granulometry BeckmanCoulter LS 13320, e.g. following ISO 13320 Particle size analysis - Laser diffraction methods.

It is furthermore preferred that (C) fulfills one or more, preferably all, of the following requirements:
- a bulk density according to ASTM B 329 in the range of from 0.2 to 1.3, preferably 0.4 to 1.1, most preferably 0.5 to 1.0 g/cm³,
- a tap density according to DIN/ISO 3953 in the range of from 0.7 to 2.3, preferably 0.9 to 2.1, most preferably 1.0 to 2.0 g/cm³, and
- a BET specific surface of 10 m²/g or less, preferably 5 m²/g or less, more preferably 3 m²/g or less, even more preferably 2 m²/g or less, and preferably 0.01 m²/g or more, more preferably 0.1 m²/g or more, as measured using BET BeckmanCoulter SA 3100.

The silver content of the silver particles is not particularly limited but is preferably high. Thus, the silver particles preferably contain at least 98 wt.-% silver, more preferably at least 99 wt.-% silver, even more preferably at least 99.2 wt.-% silver, more preferably at least 99.5 wt.-% silver, more preferably at least 99.8 wt.-% silver, more preferably at least 99.9 wt.-% silver, relative to the total weight of the silver particles.

The silver particles may be an alloy of silver and copper, wherein the silver particles typically contain 100 ppm to 600 wt.-ppm, preferably 200 to 500 wt.-ppm, more preferably 250 to 450 wt.-ppm copper, relative to the total weight of the silver particles.

It is preferred that the silver particles are agglomerated silver particles. Thus, primary particles of silver are preferably agglomerated to form secondary particles.

The silver particles are preferably obtained by chemical precipitation. The preparation of silver particles with controlled morphologies and particle sizes is known to the skilled person and, e.g., described "Precipitation of silver particles with controlled morphologies from aqueous solutions" by Wang et all in CrystEngComm 2020 (https://doi.org/10.1039/C9CE01601E).

Examples of suitable silver particles as component (C) include, but are not limited to, "Silver-Powder 327085" by Sigma Aldrich, "Ag Spherical 41597" by AlfaAesar, "Ag Spherical 41599" by AlfaAesar, or "Silberflakes F56", "Silberflakes B190" or "Silberpulver AGP H1080-1" by Doduco.

### The thermoplastic elastomer containing essentially no groups derived from a dicarboxylic acid anhydride containing an aliphatic C=C double bond (D)

Component (D) is preferably a block copolymer. More preferably, component (D) is a block copolymer of styrene with one or more selected from ethylene, butylene, propylene and isoprene. Component (D) is more preferably a block copolymer of styrene and butylene, optionally with one or both selected from propylene and isoprene.

It is to be understood that the Component (D) can also be a partially hydrogenated styrene-block-copolymer. The hydrogenation preferably refers to the hydrogenation of aliphatic C=C double bonds, but not aromatic C=C double bonds. In view of oxidation stability, it is preferred that at least 50 %, preferably at least 60 %, more preferably at least 70 %, even more preferably at least 80 %, or even at least 90 % of all aliphatic C=C double bonds are hydrogenated. In other words, hydrogenated block copolymers typically have a residual aliphatic double bond content of 0.5 to 20%, based on the content present before hydrogenation. Typically less than 20 mol-%, preferably less than 12 mol-%, more preferably less than 5 mol-%, even more preferably less than 3 mol-%, still more preferably less than 2 mol-%, less than 1 mol-%, or even less than 0.5 mol-% of the aliphatic carbon atoms in component (D) are carbon atoms forming a C=C double bond.

As used herein, the term styrene-ethylene-butylene-styrene (SEBS), preferably includes any hydrogenated or partially hydrogenated styrene/butadiene-styrene (SBS) copolymers.

Component (D) is preferably a styrene-block-copolymer or a hydrogenated styrene-block-copolymer, obtainable by
(i) polymerizing
   - styrene, and
   - at least one selected from ethylene, butylene, propylene and isoprene, and
(ii) optionally hydrogenating the copolymer.

Component (D) is a thermoplastic elastomer containing essentially no groups derived from a dicarboxylic acid anhydride containing an aliphatic C=C double bond. The expression "containing essentially no groups derived from a dicarboxylic acid anhydride containing an aliphatic C=C double bond" means that (D) typically contains less than 0.1 wt.-%, preferably less than 0.05 wt.-%, more preferably less than 0.01 %, even more preferably less than 0.001 wt.-% groups derived from a dicarboxylic acid anhydride containing an aliphatic C=C double bond, relative to the total weight of (D).

(D) is preferably a styrene-block-copolymer or a hydrogenated styrene-block-copolymer, obtainable by polymerizing styrene and at least one selected from ethylene, butylene, propylene and isoprene and optionally hydrogenating the copolymer. More preferably, (D) is a styrene-block-copolymer selected from styrene-ethylene-butylene-styrene (SEBS), styrene/butadiene-styrene (SBS), styrene-ethylene/propylene-styrene (SEPS), styrene-isoprene-styrene (SIS), or a hydrogenated copolymer of these.

Component (D) preferably fulfills one or more, preferably all, of the following requirements:
- a hardness (4 mm, 15 s) measured according to ASTM D 2240 of in the range of 20 to 70 Shore A, preferably 30 to 60 Shore A, more preferably 35 to 60 Shore A, even more preferably 35 to 52 Shore A, most preferably 35 to 45 Shore A,
- a specific gravity measured according to ASTM D 792 in the range of 0.88 to 0.90 g/cm³,
- a tensile strength measured according to ASTM D 638 in the range of 4.0 to 13.0 MPa,
- a tear strength measured according to ASTM D 624 in the range of 11.0 to 37.0 kN/m,
- a stress at 100% strain measured according to ASTM D 638 in the range of 0.4 to 2.4 MPa,
- a stress at 300% strain measured according to ASTM D 638 in the range of 0.9 to 3.5 MPa,
- a elongation at break measured according to ASTM D 638 in the range of 600% to 800 %, and
- a melt flow rate measured according to ASTM D 1238 at 190°C/5.0 kg in the range of 0.5 to 20.0 g/10 min, preferably in the range of 0.5 to 15.0 g/10 min, more preferably in the range of 0.5 to 10.0 g/10 min, even more preferably in the range of 0.5 to 5.0 g/10 min.

### The method of manufacturing the composition

The present disclosure also relates to a method of manufacturing the composition of the present disclosure. The method is not particularly limited as long as the composition of the present disclosure is thereby obtained. The method preferably comprises the steps of:
- a step of providing molten (A) and optionally (D), and
- a step of adding (preferably slowly) a mixture of (B) and (C).

In particular if no (D) is used, the method preferably comprises the steps of:
- a step of providing molten (A), and
- a step of adding a mixture of (B) and (C) and optionally further (A).

For example, A and optionally D can be fed and melted with two gravimetric balances in hopper 1 in a Buss Kneader, and then B and C can be added slowly with two gravimetric balances in hopper 2. The material can then be cooled, preferably with water cooling, and then pelletized and dried.

(A), (B), (C) and optionally (D) may be compounded using an extruder or kneader, such as a double screw extruder or a buss kneader, preferably at low to moderate shear, such as at 100-200 rpm in the mixing zone of a Buss Kneader.

In principle, any standard industrial process/machine suitable for the fabrication of highly filled compound systems, e.g. a double rolling mill, internal mixer, single- or twin screw extruder, Buss kneader or a comparable processing machine can be used for the production of the composition.

### The molded article

The present disclosure also relates to a molded article obtainable by molding the composition according to the present disclosure.

The molded article is preferably a sensor or an electrode. In the present disclosure, the molded article can be in the form of any device commonly used in contact with human skin. Preferred examples of the molded article include a pad or an eartip, or part of an eartip, earclip, headphones (e.g. in-ear or over-ear headphones), watch, ring, glasses, underwear, belt, hat, shirt, chest strap, headband, helmet, glove, socks, shoe, mouse, game controller, steering wheel (such as for a car, truck, train, plane or boat), remote control (such as for a TV, drone, car or boat), bioimpedance scale.

The molded article, e.g. in the form of a sensor or an electrode, can be used for measuring an electrophysiological signal, preferably a bioelectrical signal, preferably a biopotential signal, preferably a electromyographic signal, preferably an electroencephalographic signal and/or electrocardiogram and/or an electrooculographic signal and/or a galvanic skin response and/or an electrooculogram and/or a body temperature and/or a pulse and/or a blood pressure and/or a body movement.

The molded article, described herein is suitable for large-scale and cost-efficient production of reusable biopotential electrodes, that are soft and flexible, thereby providing outstanding user comfort while at the same time offering the advantage of very low skin contact impedance that is a prerequisite for high signal quality of acquired data. These facts open the door to functionalizing common everyday articles and electronic devices with biopotential recording capabilities in an unobtrusive manner. Articles and devices equipped with molded articles described in the present disclosure are comparable to non-functionalized articles and devices optically and in terms of user comfort.

The molded article is preferably associated with a processor and/or a memory for processing the measured signals.

Methods for preparing the molded article are not particularly limited. For example, the molded article or electronic device is obtainable by one or more selected from injection molding, overmoulding, vacuum molding, vaccum forming, thermoforming, 3D printing such as 3D printing directly onto textiles or other substrates, flat dye extrusion optionally followed by punching, laser cutting or water jet cutting into desired shape, melt coating such as melt coating on textiles, films, non-woven, transfer coating followed by lamination onto a substrate. The molded article is preferably injection-molded.

### The electronic device

The present disclosure furthermore concerns an electronic device comprising the molded article according to the present disclosure. For example, the electronic device may contain a part which is a molded article according to the disclosure in addition to other parts which are made of commonly used materials such as polymeric materials or metals. In a watch, as an example of an electronic device, the molded article according to the present disclosure may form part of the watch casing which is in contact with skin, while the other parts of the watch casing may be of other materials. Alternatively, the molded article according to the present disclosure may form part of the watch strap whereas the watch casing may be a usual watch casing.

The electronic device is preferably for measuring an electrophysiological signal, preferably a bioelectrical signal, preferably a biopotential signal, preferably a electromyographic signal, preferably an electroencephalographic signal and/or electrocardiogram and/or an electrooculographic signal and/or a galvanic skin response and/or an electrooculogram and/or a body temperature and/or a pulse and/or a blood pressure and/or a body movement.

The electronic device preferably comprises a processor and/or a memory for processing the measured signals.

The electronic device is obtainable by one or more selected from injection molding, overmoulding, vacuum molding, vacuum forming, thermoforming, 3D printing such as 3D printing directly onto textiles or other substrates, flat dye extrusion optionally followed by punching, laser cutting or water jet cutting into desired shape, melt coating such as melt coating on textiles, films, non-woven, transfer coating followed by lamination onto a substrate.

### The use of the molded article or electronic device

The molded article or the electronic device according to the present disclosure is preferably used in one or more selected from an electrocardiogram (ECG), an electromyogram (EMG), an electroencephalogram (EEG), a galvanic skin response (GSR), an electrooculogram (EOG), a body temperature, a pulse, a blood pressure, a body movement.

Typically, the molded article or electronic device is used for measuring an electrophysiological signal, preferably a bioelectrical signal, preferably a biopotential signal, preferably an electroencephalographic signal and/or electrocardiogram and/or an electrooculographic signal and/or a galvanic skin response and/or an electrooculogram and/or a body temperature and/or a pulse and/or a blood pressure and/or a body movement.

The use suitably comprises applying the molded article or electronic device to part of the skin of an animal. Two or more of the molded articles or two or more of the electronic devices are preferably used in order to enable measuring a multitude of signals. Most preferably, the molded article or electronic device are for use in measuring electric voltage.

### The method of measuring an electrophysiological signal

The molded article or electronic device according to the present disclosure may be used for measuring an electrophysiological signal by any known method. Preferably the method of measuring an electrophysiological signal includes the steps:
- preferably clean skin areas where electrode will be applied, e.g., with a dry cotton swab or paper tissue to remove any sebum, lotion or cerumen present on the surface of the skin,
- applying one or more, preferably two or more, of molded articles and/or electronic devices according to the present disclosure to part of the skin of an animal, and
- measuring electric voltage, i.e. the potential difference between the two or more areas of skin, to which the two or more electrodes are applied.
- recording, amplifying and processing the measured potential differences.

The term "animal" as used herein preferably refers to a mammal, more preferably a human.

The part of the skin of an animal is preferably skin of the ear and/or skin around the ear, more preferably skin of the pinna or ear canal, more preferably skin at the outer part of the ear canal.

The present disclosure may be summarized by the following items:
1. A composition comprising:
   (A) a thermoplastic elastomer containing groups derived from a dicarboxylic acid anhydride containing an aliphatic C=C double bond,
   (B) carbon black, and
   (C) silver particles.
2. The composition according to item 1, wherein (A) contains at least 0.1 wt.-%, preferably at least 0.2 wt.-%, more preferably at least 0.5 wt.-%, even more preferably at least 1.0 wt.-% groups derived from a dicarboxylic acid anhydride containing an aliphatic C=C double bond, relative to the total weight of (A).
3. The composition according to item 1 or 2, wherein (A) contains less than 15 wt.-%, preferably less than 10 wt.-%, more preferably less than 8.0 wt.-%, even more preferably less than 6.0 wt.-%, still even more preferably less than 4.0 wt.-%, or even less than 2.0 wt.-%, groups derived from a dicarboxylic acid anhydride containing an aliphatic C=C double bond, relative to the total weight of (A).
4. The composition according to any one of items 1 to 3, wherein (A) is a styrene-block-copolymer or a hydrogenated styrene-block-copolymer, obtainable by
   (i) polymerizing
      - styrene, and
      - at least one selected from ethylene, butylene propylene and isoprene,
   (ii) optionally hydrogenating the copolymer, and
   (iii) graft polymerization of the copolymer or hydrogenated copolymer with a dicarboxylic acid anhydride containing an aliphatic C=C double bond.
5. The composition according to any one of items 1 to 4, wherein the carboxylic acid anhydride containing an aliphatic C=C double bond is maleic acid anhydride.
6. The composition according to any one of items 1 to 5, wherein (A) is a maleic acid anhydride modified styrene-block-copolymer selected from maleic acid anhydride modified styrene-ethylene-butylene-styrene (SEBS), maleic acid anhydride modified styrene/butadiene-styrene (SBS), maleic acid anhydride modified styrene-ethylene/propylene-styrene (SEPS), maleic acid anhydride modified styrene-isoprene-styrene (SIS), or a hydrogenated copolymer of these.
7. The composition according to any one of items 1 to 6, wherein (A) is a styrene-ethylene-/butylene-styrene-block-copolymer, preferably a styrene-ethylene-/butylene-styrene-block-copolymer which has been modified with maleic acid anhydride, such as a copolymer obtainable by polymerization at least styrene, maleic acid anhydride and ethylene or butylene.
8. The composition according to any one of items 1 to 7, wherein (B) has a BET surface in the range of from 10 to 1500 m²/g, preferably 10 to 1000 m²/g, more preferably 20 to 900 m²/g, even more preferably 25 to 600 m²/g, still more preferably 25 to 400 m²/g, even still more preferably 30 to 200 m²/g, most preferably 30 to 100 m²/g or even 50 to 90 m²/g, as measured according to ASTM D3037-89.
9. The composition according to any one of items 1 to 8, wherein (B) fulfills one or more, preferably all, of the following requirements:
   - a pour density measured according to ASTM D1513 of 130 to 210, preferably 150 to 190, more preferably 160 to 180 g/l,
   - an oil absorption number measured according to ASTM D2414 of 170 to 210, preferably 180 to 200, more preferably 185 to 195 ml/100g, and
   - a grit 325 mesh residue according to ASTM D1514 of 50 wt.-ppm or less, preferably 20 wt.-ppm or less, more preferably 10 wt.-ppm or less, even more preferably 5 wt.-ppm or less.
10. The composition according to any one of items 1 to 9, wherein (C) have a median particle diameter in the range of from 0.1 to 20 µm, preferably 0.2 to 15 µm, more preferably 0.3 to 13 µm, even more preferably 0.4 to 12 µm, still more preferably 0.5 to 10 µm, even still more preferably 1 to 10 µm, most preferably 2 to 9 µm, as measured by laser granulometry, preferably using laser granulometry BeckmanCoulter LS 13320.
11. The composition according to any one of items 1 to 10, wherein (C) fulfills one or more, preferably all, of the following requirements:
   - a bulk density according to ASTM B 329 in the range of from 0.2 to 1.3, preferably 0.4 to 1.1, most preferably 0.5 to 1.0 g/cm³,
   - a tap density according to DIN/ISO 3953 in the range of from 0.7 to 2.3, preferably 0.9 to 2.1, most preferably 1.0 to 2.0 g/cm³, and
   - a BET specific surface of 10 m²/g or less, preferably 5 m²/g or less, more preferably 3 m²/g or less, even more preferably 2 m²/g or less, and preferably 0.01 m²/g or more, more preferably 0.1 m²/g or more, as measured using BET BeckmanCoulter SA 3100.
12. The composition according to any one of items 1 to 11, wherein the silver particles contain at least 98 wt.-% silver, more preferably at least 99 wt.-% silver, even more preferably at least 99.2 wt.-% silver, more preferably at least 99.5 wt.-% silver, more preferably at least 99.8 wt.-% silver, more preferably at least 99.9 wt.-% silver, relative to the total weight of the silver particles.
13. The composition according to any one of items 1 to 12, wherein the silver particles contain 100 ppm to 600 wt.-ppm, preferably 200 to 500 wt.-ppm, more preferably 250 to 450 wt.-ppm copper, relative to the total weight of the silver particles.
14. The composition according to any one of items 1 to 13, wherein the silver particles are agglomerated silver particles.
15. The composition according to any one of items 1 to 14, wherein the silver particles are obtainable by chemical precipitation.
16. The composition according to any one of items 1 to 15, further comprising (D) a thermoplastic elastomer containing essentially no groups derived from a dicarboxylic acid anhydride containing an aliphatic C=C double bond.
17. The composition according to any one of items 1 to 16, wherein (D) contains less than 0.1 wt.-%, preferably less than 0.05 wt.-%, more preferably less than 0.01 %, even more preferably less than 0.001 wt.-% groups derived from a dicarboxylic acid anhydride containing an aliphatic C=C double bond, relative to the total weight of (A).
18. The composition according to any one of items 1 to 17, wherein (A) is a styrene-block-copolymer or a hydrogenated styrene-block-copolymer, obtainable by polymerizing styrene and at least one selected from ethylene, butylene, propylene and isoprene, optionally hydrogenating the copolymer and functionalizing the copolymer with a dicarboxylic acid anhydride containing an aliphatic C=C double bond.
19. The composition according to any one of items 1 to 18, wherein (A) is obtainable by reacting the copolymer with a dicarboxylic acid anhydride containing an aliphatic C=C double bond and optionally an initiator, such as benzoyl peroxide or azobisisobutyronitrile, preferably at a temperature of 40 to 100°C, more preferably 50 to 90°C, preferably using solid phase grafting using a twin-screw device, such as high-sheer solid phase grafting, typically as set out in WO 93/24537.
20. The composition according to any one of items 1 to 19, wherein (D) is a styrene-block-copolymer selected from styrene-ethylene-butylene-styrene (SEBS), styrene/butadienestyrene (SBS), styrene-ethylene/propylene-styrene (SEPS), styrene-isoprene-styrene (SIS), or a hydrogenated copolymer of these.
21. The composition according to any one of items 1 to 20, wherein (D) fulfills one or more, preferably all, of the following requirements:
   - a hardness (4 mm, 15 s) measured according to ASTM D 2240 of in the range of 20 to 70 Shore A, preferably 30 to 60 Shore A, more preferably 35 to 60 Shore A, even more preferably 35 to 52 Shore A, most preferably 34 to 45 Shore A,
   - a specific gravity measured according to ASTM D 792 in the range of 0.88 to 0.90 g/cm³,
   - a tensile strength measured according to ASTM D 638 in the range of 4.0 to 13.0 MPa,
   - a tear strength measured according to ASTM D 624 in the range of 11.0 to 37.0 kN/m,
   - a stress at 100% strain measured according to ASTM D 638 in the range of 0.4 to 2.4 MPa,
   - a stress at 300% strain measured according to ASTM D 638 in the range of 0.9 to 3.5 MPa,
   - a elongation at break measured according to ASTM D 638 in the range of 600% to 800 %, and
   - a melt flow rate 190°C/5.0 kg measured according to ASTM D 1238 in the range of 0.5 to 20.0 g/10 min, preferably in the range of 0.5 to 15.0 g/10 min, more preferably in the range of 0.5 to 10.0 g/10 min, even more preferably in the range of 0.5 to 5.0 g/10 min.
22. The composition according to any one of items 1 to 21, wherein the composition contains (D) in a range of from 5 to 50 wt-%, preferably 5 to 30 wt-%, more preferably 10 to 30 wt.-%, even more preferably 10 to 25 wt.-%, still more preferably 12 to 24 wt.-%, most preferably 13 to 24 wt.-%, such as 13 wt.-%, 14 wt.-%, 15 wt.-%, 16 wt.-%, 17 wt.-%, 18 wt.-%, 19 wt.-%, 20 wt.-%, 21 wt.-%, 22 wt.-%, 23 wt.-%, or 24 wt.-%, relative to 100 wt.-% of the composition.
23. The composition according to any one of items 1 to 22, wherein the composition contains (A) in a range of from 5 to 50 wt-%, preferably 8 to 40 wt-%, more preferably 10 to 30 wt.-%, even more preferably 15 to 25 wt.-%, still more preferably 17 to 23 wt.-%, most preferably 19 to 21 wt.-%, such as 19 wt.-%, 20 wt.-%, or 21 wt.-%, relative to 100 wt.-% of the composition.
24. The composition according to any one of items 1 to 23, wherein the composition contains (B) in a range of from 1 to 40 wt-%, preferably 2 to 35 wt-%, more preferably 3 to 30 wt.-%, even more preferably 5 to 30 wt.-%, still more preferably 5 to 25 wt.-%, still even more preferably 8 to 20 wt.-%, most preferably 10 to 20 wt.-%, such as 10 wt.-%, 11 wt.-%, 12 wt.-%, 13 wt.-%, 14 wt.-%, 15 wt.-%, 16 wt.-%, 17 wt.-%, 18 wt.-%, 19 wt.-%, or 20 wt.-%, relative to 100 wt.-% of the composition.
25. The composition according to any one of items 1 to 24, wherein the composition contains (C) in a range of from 10 to 90 wt-%, preferably 20 to 70 wt-%, more preferably 25 to 65 wt.-%, even more preferably 30 to 60 wt.-%, still more preferably 36 to 60 wt.-%, still even more preferably 36 to 57 wt.-%, such as 36 wt.-%, 37 wt.-%, 38 wt.-%, 39 wt.-%, 40 wt.-%, 41 wt.-%, 42 wt.-%, 43 wt.-%, 44 wt.-%, 45 wt.-%, 46 wt.-%, 47 wt.-%, 48 wt.-%, 49 wt.-%, 50 wt.-%, 51 wt.-%, 52 wt.-%, 53 wt.-%, 54 wt.-%, 55 wt.-%, 56 wt.-%, or 57 wt.-%, relative to 100 wt.-% of the composition, wherein particularly preferred are 40 wt.-%, 41 wt.-%, 42 wt.-%, 43 wt.-%, 44 wt.-%, 45 wt.-%, 46 wt.-%, 47 wt.-%, 48 wt.-%, 49 wt.-%, or 50 wt.-%; more preferred are 42 wt.-%, 43 wt.-%, 44 wt.-%, 45 wt.-%, 46 wt.-%, or 47 wt.-%, such as 43 wt.-%, 44 wt.-%, 45 wt.-%, or 46 wt.-%.
26. The composition according to any one of items 1 to 25, wherein the ratio of (B) to (C) (each in wt.-%) is in the range of from 0.01 to 50, preferably 0.05 to 20, more preferably 0.1 to 10, even more preferably 0.2 to 5, still more preferably 0.2 to 2, even still more preferably 0.2 to 1.
27. The composition according to any one of items 1 to 26, wherein the total content of (D), (A), (B) and (C) is at least 70 wt.-%, preferably at least 75 wt.-%, more preferably at least 80 wt.-%, even more preferably at least 90 wt.-%, still more preferably at least 95 wt.-%, or even at least 96 wt.-%, at least 97 wt.-%, at least 98 wt.-%, at least 99 wt.-%, at least 99.5 wt.-%, or at least 99.9 wt.-%, relative to 100 wt.-% of the composition.
28. A method of manufacturing the composition according to any of items 1 to 27, comprising:
   - a step of providing molten (A) and optionally (D), and
   - a step of adding (preferably slowly) a mixture of (B) and (C).
29. The method according to item 28, further comprising the step of compounding using an extruder or kneader, such as a double screw extruder or a buss kneader, preferably at low to moderate shear, such as at 100-200 rpm, preferably 160-200 rpm, in the mixing zone of a Buss Kneader.
30. A molded article obtainable by molding the composition according to any one of items 1 to 28.
31. The molded article according to item 30, wherein the molded article is injection-molded.
32. The molded article according to item 30 or 31, being a sensor or an electrode.
33. The molded article according to any one of items 30 to 32, wherein the molded article is in the form of a pad or an eartip, or part of an eartip (including any form of stabilizing structure that helps to stabilize earbuds in the ear, e.g. in the concha of the ear), earclip, watch, ring, glasses, underwear, belt, hat, shirt, chest strap, headband, helmet, glove, socks, shoe, mouse, game controller, steering wheel (such as for a car, truck, train, plane or boat), remote control (such as for a TV, drone, car or boat), bioimpedance scale, or any other device commonly being used in contact with human skin.
34. The molded article according to any one of items 30 to 33, being a sensor or an electrode for measuring an electrophysiological signal, preferably a bioelectrical signal, preferably a biopotential signal, preferably a electromyographic signal, preferably an electroencephalographic signal and/or electrocardiogram and/or an electrooculographic signal and/or a galvanic skin response and/or an electrooculogram and/or a body temperature and/or a pulse and/or a blood pressure and/or a body movement.
35. The molded article according to any one of items 30 to 34, wherein the molded article or electronic device is obtainable by one or more selected from injection molding, overmoulding, vacuum molding, vacuum forming, thermoforming, 3D printing such as 3D printing directly onto textiles or other substrates, flat dye extrusion optionally followed by punching, laser cutting or water jet cutting into desired shape, melt coating such as melt coating on textiles, films, non-woven, transfer coating followed by lamination onto a substrate.
36. An electronic device comprising the molded article according to any one of items 30 to 35.
37. The electronic device according to item 36, wherein the electronic device is for measuring an electrophysiological signal, preferably a bioelectrical signal, preferably a biopotential signal, preferably a electromyographic signal, preferably an electroencephalographic signal and/or electrocardiogram and/or an electrooculographic signal and/or a galvanic skin response and/or an electrooculogram and/or a body temperature and/or a pulse and/or a blood pressure and/or a body movement.
38. The electronic device according to item 36 or 37, wherein the molded article or electronic device is obtainable by one or more selected from injection molding, overmoulding, vacuum molding, vacuum forming, thermoforming, 3D printing such as 3D printing directly onto textiles or other substrates, flat dye extrusion optionally followed by punching, laser cutting or water jet cutting into desired shape, melt coating such as melt coating on textiles, films, non-woven, transfer coating followed by lamination onto a substrate.
39. Use of the molded article according to any one of items 30 to 35 or the electronic device according to any one of items 36 to 38, in one or more selected from an electrocardiogram (ECG), an electromyogram (EMG), an electroencephalogram (EEG), a galvanic skin response (GSR), an electrooculogram (EOG), a body temperature, a pulse, a blood pressure, a body movement.
40. Use of the molded article according to any one of items 30 to 35 or the electronic device according to any one of items 36 to 38, for measuring an electrophysiological signal, preferably a bioelectrical signal, preferably a biopotential signal, preferably an electroencephalographic signal and/or electrocardiogram and/or an electrooculographic signal and/or a galvanic skin response and/or an electrooculogram and/or a body temperature and/or a pulse and/or a blood pressure and/or a body movement.
41. The use according to item 39 or 40, wherein the use comprises applying the molded article or electronic device to part of the skin of an animal.
42. The use according to any one of items 39 to 41, wherein the use encompasses two or more of the molded articles or two or more of the electronic devices.
43. The use according to any one of items 39 to 42, wherein the use comprises measuring electric voltage.
44. A method of measuring an electrophysiological signal including the steps:
   - applying one or more, preferably two or more, of molded articles according to any one of items 30 to 35 or electronic devices according to any one of items 36 to 38 to part of the skin of an animal, and
   - measuring electric voltage.
45. The use according to any of items 39 to 43 or the method according to item 44, wherein the animal is a mammal, preferably a human.
46. The use according to any of items 39 to 43 or 45, or the method according to item 44 or 45, wherein the part of the skin of an animal is skin of the ear, preferably skin of the pinna or ear canal, more preferably skin of the outer part of the ear canal.

The following examples are meant to illustrate the present disclosure. The examples are presented to exemplify the disclosure and are not to be considered as limiting the scope of the present disclosure.

While various embodiments of the present disclosure have been described above, it should be understood that they have been presented by way of example only, and not by way of limitation. Likewise, any diagrams may depict an example architecture or configuration, which are provided to enable persons of ordinary skill in the art to understand exemplary features and functions of the present disclosure. Such persons would understand, however, that the present disclosure is not restricted to the illustrated example architectures or configurations, but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any one of the above-described exemplary embodiments.

It is also understood that any reference to an element herein using a designation such as "first", "second", and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner.

Additionally, a person having ordinary skill in the art would understand that information and signals can be represented using any one of a variety of different technologies and techniques. For example, data, instructions, commands, information, signals, bits and symbols, for instance, which may be referenced in the above description can be represented by voltages, currents, charges, electromagnetic waves, (electro-)magnetic fields or particles, optical fields or particles, or any combination thereof. Certain blocks, units, etc. may also convert/transform signals etc. from one representation to another

A skilled person would further appreciate that any one of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions and/or configurations (which can be referred to herein, for convenience, as "software" or a "software unit"), or any combination of these techniques.

To clearly illustrate this interchangeability of hardware, firmware and software, various illustrative components, blocks, units, circuits, and steps have been described above generally in terms of their functionality (e.g. the molded article and/or the electronic device). Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed and/or programmed and/or arranged to perform the specified operation or function.

Furthermore, a skilled person would understand that various illustrative logical blocks, units, devices, components and circuits described herein can be implemented within or performed by an integrated circuit (IC) that can include a general purpose processor, a digital signal processor (DSP), an application specific integrated circuit (ASIC), a field programmable gate array (FPGA) or other programmable logic device, or any combination thereof. The logical blocks, units, and circuits can further include antennas and/or transceivers to communicate with various components within the network or within the device. A general purpose processor can be a microprocessor, but in the alternative, the processor can be any conventional processor, controller, or state machine. A processor can also be implemented as a combination of computing devices, e.g., a combination of a DSP and a microprocessor, a plurality of microprocessors, one or more microprocessors in conjunction with a DSP core, or any other suitable configuration to perform the functions described herein. If implemented in software, the functions can be stored as one or more instructions or code or configuration on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

In this document, the term "unit" as used herein, refers to software, firmware, hardware, and any combination of these elements for performing the associated functions described herein. Additionally, for purpose of discussion, the various units are described as discrete units; however, as would be apparent to one of ordinary skill in the art, two or more units may be combined to form a single unit that performs the associated functions according to embodiments of the present disclosure.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure. For example, functionality illustrated to be performed by separate processing logic elements, or controllers, may be performed by the same processing logic element, or controller. Hence, references to specific functional units are only references to a suitable means for providing the described functionality, rather than indicative of a strict logical or physical structure or organization.

Various modifications to the implementations described in this disclosure will be readily apparent to those skilled in the art, and the general principles defined herein can be applied to other implementations without departing from the scope of this disclosure. Thus, the disclosure is not intended to be limited to the implementations shown herein, but is to be accorded the widest scope consistent with the novel features and principles disclosed herein, as recited in the claims below.

### Examples

### Example 1

### Materials

SCONA TSKD 9103 (BYK-Chemie GmbH), as component (A)
Ensaco 260G (Imerys S.A.), as component (B)
Silberpulver AGP H1080-1 (Doduco GmbH), as component (C)
Mediprene 500400M-03 (Hexpol TPE), as component (D)
Mediprene 500520M-03 (Hexpol TPE), as component (D)

### Experimental

### Lab scale production of the compositions and fabrication of prototypes

For small-scale compounding in the lab of components (A), (B), (C) and (D) a Haake Polylab OS RhepDrive was used for mixing the components to a homogeneous mixture. The mixing chamber of 69cm³ was used with a maximum filling level of 70% (48cm³). Roller rotors were used for compounding to achieve a homogeneous compound. The Haake Polylab was always preheated to 180°C and the temperature during mixing was 200°C. A shear rate of 10 rpm was used to melt the component (A) and (D) and was increased afterwards to 30 rpm to introduce stepwise the components (B) and (C). The mixing time after introducing the components to the Haake Polylab OS RheoDrive was 30 min.

After compounding, the fabrication to 3D-shaped prototypes was done with the capillary rheometer Flowmaster RH7. The material was extruded at 180°C with a speed of 5 mm/min. The extrusion pressure was detected by a 1400 bar pressure sensor. The extrusion to a 3D-shaped electrode was done with an eartip-shaped steel mold that can be used in the capillary rheometer.

### Large-scale production of the composition

For large-scale compounding of the composition, appropriate amounts (see Table 1) of SCONA TSKD 9103 and Mediprene 500400M-03 or Mediprene 500520M-03 were dosed separately with two different gravimetric balances into the first inlet of a Buss Kneader. In the second inlet, an appropriate amount of Ensaco 260G as well as of Silberpulver AGP H1080-1 (see Table 1 was dosed with two separate gravimetric balances. The temperature of kneading zone one was set to 200 - 220°C. Kneading zone two was situated between the last inlet and the discharge extruder of the buss kneader and was heated to 190 - 210°C and had a speed of 120 to 200 rpm. The discharge extruder was heated to 130 - 180°C, the die of the kneader was heated to around 190°C. After passing the die, at a rate of 8 to 20 kg/h, the mixed compound was cooled with water to facilitate pelletization. In a final step, the pellets were dried before they were packaged into airtight bags that also protect the compound from UV light.

**Table 1**

| Raw Material | Example 1-1 | Example 1-2 | Example 1-3 | Example 1-4 | Example 1-5 | Example 1-6 |
|---|---|---|---|---|---|---|
| SCONA TSKD 9103 | 20.0 wt% | 20.0 wt% | 20.0 wt% | 20.0 wt% | 20.0 wt% | 20.0 wt% |
| Ensaco 260G | 16.0 wt% | 16.0 wt% | 20.0 wt% | 20.0 wt% | 10.2 wt% | 10.2 wt% |
| Silberpulver AGP H1080-1 | 44.5 wt% | 44.5 wt% | 36.7 wt% | 36.7 wt% | 56.4 wt% | 56.4 wt% |
| Mediprene 500400M-03 | 19.5 wt% | - | 23.3 wt% | - | 13.4 wt% | - |
| Mediprene 500520M-03 | - | 19.5 wt% | - | 23.3 wt% | - | 13.4 wt% |

### Large scale fabrication of the molded article from the composition

Composition material from Example 1-1, 1-2, 1-3, 1-4, 1-5 or 1-6 was used for production of the molded article by injection molding.
The temperature in the screw of the injection molding machine was set to between 40°C in the feeder part of the screw to up to 270°C in the part of the screw closest to the nozzle, the nozzle temperature being set to up to 270°C. The mold itself was operated between 30°Cand 80°C. The injection pressure was set between 1000 bar to 1800 bar, with an after-pressure of 400 to 1000 bar. After filling the mold, the part was cooled in the mold for 5 to 30 seconds before it was ejected.

In such a manner, up to thousands of molded articles can be produced per day, allowing for a highly reproducible, scalable, and cost-effective way of production for highly performant dry in-ear EEG sensors.

### Results and Evaluation

### Characterization of the molded articles made from the composition

Molded articles, fabricated as described from the composition from Examples 1-1, 1-2, 1-3, 1-4, 1-5 and 1-6 were characterized with regards to skin-contact impedance in the ear canal. For each one of the Examples 1-1, 1-2, 1-3, 1-4, 1-5 and 1-6, six measurements were conducted on each subject (three in the left and three in the right ear) between 0.1 to 120 Hz, with a current range of 0.1 to 10 µA. It is immediately visible in Fig. 1a and b, that all of the examples have a similarly low impedance in the ear canal, i.e. 150 kOhm or less across 0.1 to 120 Hz. It is generally known from the literature, that a low skin-electrode impedance is a major prerequisite for a robust EEG signal quality *(*Sensors 2021, 21(15), 5210; https://doi.org/10.3390/s21155210). Slightly higher impedance values were recorded for Example 1-5 and 1-6, while Examples 1-1, 1-2, 1-3 and 1-4 perform very similar. For a better reproducibility of impedance values measured on human skin, in this case in the outer ear canal, it is very important to clean the ear canal from any cerumen or lotion products and to strictly adhere to a 10 minute long stablilization time of the molded articles inserted into the outer ear canal before the start of the impedance measurement.

### Example 2

### Influence of Component A on mechanical and electrical properties of the composition

The elongation at the point of fracture of compositions from Examples 1-1, 2-1, 2-2 and 2-3 (see Table 2) was measured according to the description, using extruded fibers with a diameter of 0.5 mm and a gauge length of 50 mm to quantity the influence of the amount of component (A) present on the mechanical stability of the afore mentioned compositions. The electrical resistivity of Examples 1-1, 2-1, 2-2 and 2-3 was measured to understand the influence of the amount of added SCONA TSKD 9103 on the electrical properties of the compositions. Mechanical results of Examples 1-1, 2-1, 2-2 and 2-3 are summarized in Table 3 and depicted in Fig. 2.

**Table 2**

| Raw Material | Example 1-1 | Example 2-1 | Example 2-2 | Example 2-3 |
|---|---|---|---|---|
| SCONA TSKD 9103 | 20.0 wt% | 15.0 wt% | 10.0 wt% | 0.0 wt% |
| Ensaco 260G | 16.0wt% | 16.0 wt% | 16.0wt% | 16.0 wt% |
| Silberpulver AGP H1080-1 | 44.5wt% | 44.5 wt% | 44.5 wt% | 44.5 wt% |
| Mediprene 500400M-03 | 19.5 wt% | 24.5 wt% | 29.5 wt% | 39.5 wt% |

### Results and Evaluation

**Table 3**

| | Example 1-1 | Example 2-1 | Example 2-2 | Example 2-3 |
|---|---|---|---|---|
| Point of Fracture in % | 265 | 235 | 213 | 167 |

From the results for the point of fracture, depicted in Fig. 2 and shown in Table 3, it is clear, that the addition of SCONA TSKD 9103 to the composition increases the point of fracture and therefore the mechanical stability of the composition significantly. This is very important for the final application of the molded article itself, as it is intended to be part of a consumer electronic device that will be worn regularly. The elongation at break increases with higher content of SCONA TSKD 9031, due to this, the lifetime of the molded articles is longer. A brittle material with a low point of fracture would not withstand the daily wear and tear without the occurrence of small defects, such as small cracks, that would be detrimental for the Sensor's electrical performance. In Fig. 3a and b it is shown, that the addition of SCONA TSKD 9103 does not negatively affect the skin contact impedance.

### Example 3

### Skin-Electrode Impedance: Comparison of the invention to a commercially available product

The current invention was developed in order to overcome signal quality issues with the previously used material formulation. The higher impedance in the lower frequencies (below 4 Hz) were directly correlated to higher in-ear impedance in the afore mentioned frequency range. The direct correlation of skin-contact impedance with signal quality of the EEG signal measured is well established in the literature (Sensors 2021, 21(15), 5210; https://doi.org/10.3390/s21155210).

For this comparison, we focused on a direct comparison of molded articles from Example 1-1 to molded articles made from a commercially available product (Kraiburg TPE Thermolast K TC8OEX-BLCK). The commercially available composition presumably contains TPS and a carbon-based filler. In Fig. 4a and b the added benefit of the presented invention it is immediately apparent. Compared to Example 1-1, the electrodes molded from commercially available material have a distinctly steeper slope in the low frequencies, with an impedance of around 700 kOhms at 0.1 Hz, meaning that they exhibit worse contact impedances in the highly relevant low frequency range. Example 1-1 not only shows lower impedance values of less than 100 kOhms, especially in the lower frequencies, but also demonstrates a more consistent impedance across the whole frequency range of interest for EEG applications (0.1 to 120 Hz). Such a low dependency of the impedance value from the frequency is a very sought-after feature for dry electrodes, which enables, among others, recording of signals in the delta frequency band (0.5 to 4 Hz), e.g. slow waves, which are characteristic for deep sleep phases. Additionally, it allows for a more consistent signal quality across the whole frequency range of interest.

### Description of equipment and measurement procedures

### Tensile testing for measurement of the point of fracture

The tensile test was used for the characterization of the mechanical properties of the compositions. A tensile testing machine Zwick Z005 from ZwickRoell (Germany) was equipped with a 200 N load cell and with pneumatic sample holder clamps set to 4 bar to minimize slipping of the sample. The point of fracture (PoF) was tested on extruded fibers with a diameter of 0.5 mm and a gauge length of 50 mm. After the force is set to zero, a tensile load with a cross head speed of 200 mm/min is applied to the specimen until final failure. The measured maximum strain then represents the point if fracture of the sample.

### In-ear impedance measurement

The skin-electrode impedance was measured on three human subjects for each of the examples, in left and right ear each with a PalmSens4 impedance analyzer (Palmsens B.V., Netherlands) using a three-electrode setup. As working electrode (WE), molded articles made from compositions described in Table 1 and Table 3, as well as from the commercially available Thermolast K TC8OEX-BLCK were used. The WE was located inside the outer ear canal of the left and right ear of each subject. Each subject cleaned their ears with a dry cotton bud before inserting the WE. Each WE was left in the ear to stabilize for 10 minutes prior to starting the measurement. As reference electrode (RE) and counter electrode (CE) standard gold cup electrodes with SAC2 Electrode Cream (Spes Medica Srl, Italy) were used. The RE was placed on the back of the neck, just below the hairline of each subject, the CE was placed below RE on the upper back. The distance between RE and CE was the same as the distance between WE and RE.

### Laboratory small-scale mixing and prototype production

In the lab the compounding of components (A), (B), (C) and (D) was done with the Haake Polylab OS RhepDrive, with a mixing chamber of 69cm³. The maximum filling level of the chamber was 70%, that reduced the volume to the maximum level of 48cm³. Roller rotors were used for compounding with a shear rate of 10 to 30 rpm. The temperature during mixing was between 180°C and 200°C. The duration of the mixing process after introducing the components to the Haake Polylab Os RheoDrive was 30 min.

To fabricate 3D-shaped prototypes, the compositon was extruded with the capillary rheometer Flowmaster RH7 with a pressure sensor of 1400bar. The compound was extruded at 180°C, with a speed of 5 mm/min. The extrusion to a 3D-shaped electrode was done with a mold designed for the capillary rheometer.

### Large scale production of the composition and fabrication of the molded article

A Buss Kneader, equipped with two or more heating zones, as well as two or more intakes, where material can be fed into the kneader, was used for mixing the material formulation. Furthermore, the specific amounts for each of the raw materials fed into the extruder was controlled by using two or more gravimetric balances, integrated into the each of the two or more intakes of the kneader. The Buss kneader was also equipped with a discharge extruder, a die, and a pelletizing unit.

The semi-automated or fully automated state of the art injection molding machine comprised, among other components, an inlet, an injection unit with a single- or twin screw extruder and a barrel, as well a screw motor drive, to melt and feed the pelletized composition into the clamping unit, where the mold and clamping motor drive are situated, among other components.

## Claims

1. A composition comprising:
(A) a thermoplastic elastomer containing groups derived from a dicarboxylic acid anhydride containing an aliphatic C=C double bond,
(B) carbon black, and
(C) silver particles.

2. The composition according to claim 1, wherein (A) contains at least 0.1 wt.-%, preferably at least 0.2 wt.-%, more preferably at least 0.5 wt.-%, even more preferably at least 1.0 wt.-% groups derived from a dicarboxylic acid anhydride containing an aliphatic C=C double bond, relative to the total weight of (A) and wherein (A) contains less than 15 wt.-%, preferably less than 10 wt.-%, more preferably less than 8.0 wt.-%, even more preferably less than 6.0 wt.-%, still even more preferably less than 4.0 wt.-%, or even less than 2.0 wt.-%, derived from a dicarboxylic acid anhydride containing an aliphatic C=C double bond, relative to the total weight of (A).

3. The composition according to claim 1 or 2, wherein (A) is a maleic acid anhydride modified styrene-block-copolymer selected from maleic acid anhydride modified styrene-ethylene-butylene-styrene (SEBS), maleic acid anhydride modified styrene/butadiene-styrene (SBS), maleic acid anhydride modified styrene-ethylene/propylene-styrene (SEPS), maleic acid anhydride modified styrene-isoprene-styrene (SIS), or a hydrogenated copolymer of these.

4. The composition according to any one of claims 1 to 3, wherein (B) has a BET surface in the range of from 10 to 1500 m²/g, preferably 10 to 1000 m²/g, more preferably 20 to 900 m²/g, even more preferably 25 to 600 m²/g, still more preferably 25 to 400 m²/g, even still more preferably 30 to 200 m²/g, most preferably 30 to 100 m²/g or even 50 to 90 m²/g, as measured according to ASTM D3037-89.

5. The composition according to any one of claims 1 to 4, wherein (C) have a median particle diameter in the range of from 0.1 to 20 µm, preferably 0.2 to 15 µm, more preferably 0.3 to 13 µm, even more preferably 0.4 to 12 µm, still more preferably 0.5 to 10 µm, even still more preferably 1 to 10 µm, most preferably 2 to 9 µm, as measured by laser granulometry, preferably using laser granulometry BeckmanCoulter LS 13320.

6. The composition according to any one of claims 1 to 5, wherein the silver particles are obtainable by chemical precipitation.

7. The composition according to any one of claims 1 to 6, further comprising (D) a thermoplastic elastomer containing essentially no groups derived from a dicarboxylic acid anhydride containing an aliphatic C=C double bond, wherein (D) contains less than 0.1 wt.-%, preferably less than 0.05 wt.-%, more preferably less than 0.01 %, even more preferably less than 0.001 wt.-% groups derived from a dicarboxylic acid anhydride containing an aliphatic C=C double bond, relative to the total weight of (D).

8. The composition according to claim 7, wherein (D) is a styrene-block-copolymer selected from styrene-ethylene-butylene-styrene (SEBS), styrene/butadiene-styrene (SBS), styrene-ethylene/propylene-styrene (SEPS), styrene-isoprene-styrene (SIS), or a hydrogenated copolymer of these.

9. The composition according to any one of claims 1 to 8, wherein the composition contains:
(A) in a range of from 5 to 50 wt-%, preferably 8 to 40 wt-%, more preferably 10 to 30 wt.-%, even more preferably 15 to 25 wt.-%, still more preferably 17 to 23 wt.-%, most preferably 19 to 21 wt.-%, such as 19 wt.-%, 20 wt.-%, or 21 wt.-%, relative to 100 wt.-% of the composition;
(B) in a range of from 1 to 40 wt-%, preferably 2 to 35 wt-%, more preferably 3 to 30 wt.-%, even more preferably 5 to 30 wt.-%, still more preferably 5 to 25 wt.-%, still even more preferably 8 to 20 wt.-%, most preferably 10 to 20 wt.-%, such as 10 wt.-%, 11 wt.-%, 12 wt.-%, 13 wt.-%, 14 wt.-%, 15 wt.-%, 16 wt.-%, 17 wt.-%, 18 wt.-%, 19 wt.-%, or 20 wt.-%, (preferably 14 wt.-%, 15 wt.-%, 16 wt.-%, 17 wt.-%, or 18 wt.-%, more preferably 15 wt.-%, 16 wt.-% or 17 wt.-%) relative to 100 wt.-% of the composition;
(C) in a range of from 10 to 90 wt-%, preferably 20 to 70 wt-%, more preferably 25 to 65 wt.-%, even more preferably 30 to 60 wt.-%, still more preferably 36 to 60 wt.-%, still even more preferably 36 to 57 wt.-%, such as 36 wt.-%, 37 wt.-%, 38 wt.-%, 39 wt.-%, 40 wt.-%, 41 wt.-%, 42 wt.-%, 43 wt.-%, 44 wt.-%, 45 wt.-%, 46 wt.-%, 47 wt.-%, 48 wt.-%, 49 wt.-%, 50 wt.-%, 51 wt.-%, 52 wt.-%, 53 wt.-%, 54 wt.-%, 55 wt.-%, 56 wt.-%, or 57 wt.-%, relative to 100 wt.-% of the composition; and
(D) in a range of from 5 to 50 wt-%, preferably 5 to 30 wt-%, more preferably 10 to 30 wt.-%, even more preferably 10 to 25 wt.-%, still more preferably 12 to 24 wt.-%, most preferably 13 to 24 wt.-%, such as 13 wt.-%, 14 wt.-%, 15 wt.-%, 16 wt.-%, 17 wt.-%, 18 wt.-%, 19 wt.-%, 20 wt.-%, 21 wt.-%, 22 wt.-%, 23 wt.-%, or 24 wt.-%, relative to 100 wt.-% of the composition.

10. The composition according to any one of claims 1 to 9, wherein the ratio of (B) to (C) (each in wt.-%) is in the range of from 0.01 to 50, preferably 0.05 to 20, more preferably 0.1 to 10, even more preferably 0.2 to 5, still more preferably 0.2 to 2, even still more preferably 0.2 to 1.

11. A molded article obtainable by molding the composition according to any one of claims 1 to 10, wherein the molded article is preferably injection molded.

12. An electronic device comprising the molded article according to claim 11.

13. The molded article according to claim 11 or the electronic device according to claim 12, being a sensor or an electrode for measuring an electrophysiological signal, preferably a bioelectrical signal, preferably a biopotential signal, preferably a electromyographic signal, preferably an electroencephalographic signal and/or electrocardiogram and/or an electrooculographic signal and/or a galvanic skin response and/or an electrooculogram and/or a body temperature and/or a pulse and/or a blood pressure and/or a body movement.

14. Use of the molded article according to claim 11 or 13 or the electronic device according to claim 12 or 13, in one or more selected from an electrocardiogram (ECG), an electromyogram (EMG), an electroencephalogram (EEG), a galvanic skin response (GSR), an electrooculogram (EOG), a body temperature, a pulse, a blood pressure, a body movement.

15. A method of measuring an electrophysiological signal including the steps:
- applying one or more, preferably two or more, of molded articles according to claim 11 or one, or more, preferably two or more, of the electronic device according to claim 12 to part of the skin of a mammal, and
- measuring electric voltage.
